# EUROPÄISCHE PATENTSCHRIFT

(11) **EP 1 090 582 B1**
(45) Veröffentlichungstag und Bekanntmachung des Hinweises auf die Patenterteilung: **23.05.2001**
(21) Anmeldenummer: 99119518.1
(22) Anmeldetag: 01.10.1999
(51) Int. Cl.: A61B 5/00, A61B 5/12

(54) **Bildgebendes Verfahren zum Ermitteln des Zustands von Gewebe**
Imaging method for determining condition of tissue
Procédé d'imagerie pour déterminer l'état de tissus

(43) Veröffentlichungstag der Anmeldung: 11.04.2001
(73) Patentinhaber: Karl Storz GmbH & Co. KG, 78532 Tuttlingen (DE)
(72) Erfinder: Irion, Klaus M., D-78576 Liptingen (DE); Beck, Gerd, D-78532 Tuttlingen (DE)
(74) Vertreter: Heuckeroth, Volker

(56) Entgegenhaltungen:
- WO-A-97/32182
- WO-A-98/55025

## Beschreibung

Die Erfindung betrifft ein bildgebendes Verfahren zum Ermitteln eines physikalischen oder chemischen Zustands von Gewebe im menschlichen oder tierischen Körper unter Verwendung von Ultraschall, bei dem zumindest ein Ultraschallimpuls im diagnostischen Frequenz- und Leistungsbereich in das Gewebe eingekoppelt und der vom Gewebe reflektierte Ultraschallechoimpuls empfangen und in Ultraschallbildverarbeitungsmitteln verarbeitet wird.

Die Erfindung betrifft ferner ein bildgebendes System zum Ermitteln eines physikalischen oder chemischen Zustands von Gewebe im menschlichen oder tierischen Körper unter Verwendung von Ultraschall, mit Ultraschallerzeugungsmitteln zum Erzeugen zumindest einen Ultraschallimpulses im diagnostischen Frequenz- und Leistungsbereich, mit Ultraschallapplikationsmitteln zum Applizieren des Ultraschallimpulses in das Gewebe, mit Ultraschallempfangsmitteln zum Empfangen des im Gewebe reflektierten Ultraschallechoimpulses, und mit Ultraschallbildverarbeitungsmitteln zum Verarbeiten des Ultraschallechoimpulses.

Ein bildgebendes Verfahren der eingangs genannten Art, das auch als Ultraschall-Echoimpuls-Verfahren bezeichnet wird, sowie ein bildgebendes System der eingangs genannten Art sind allgemein bekannt.

Bei dieser Art eines Bildgebungsverfahrens wird beispielsweise mittels eines piezoelektrischen Ultraschallwandlers ein elektrischer Impuls in einen Ultraschallimpuls umgewandelt. Dieser wird in das zu untersuchende Gewebe eingekoppelt. Beim Eindringen des Ultraschallimpulses in das Gewebe wird dieser an Gewebsgrenzflächen teilweise reflektiert und teilweise dringt der Ultraschallimpuls weiter in das Gewebe ein. Mit diesem Verfahren können daher mehrere hintereinander liegende Gewebsschichten lokalisiert und der Zustand dieser Gewebsschichten ermittelt werden.

Unter physikalischem Zustand im Sinne der vorliegenden Erfindung werden sowohl beispielsweise ortsabhängige Parameter, wie die räumliche Ausdehnung, die räumliche Lage, die Dicke des Gewebes, als auch andere physikalische Größen, wie beispielsweise die Dichte des untersuchten Gewebes in Abhängigkeit vom Ort, verstanden. Unter dem Verfahren wird aber auch einfach die bildliche Darstellung von Gewebe in einer Bilddarstellungseinheit verstanden. Unter chemischem Zustand wird beispielsweise die Zusammensetzung des Gewebes verstanden.

Mittels des Ultraschall-Echoimpuls-Verfahrens können Gewebe von Organen bildlich dargestellt werden und Informationen über das Gewebe gewonnen werden, beispielsweise durch Auswertung des Ultraschallbildes kann ein pathologischer Zustand des Gewebes festgestellt werden. Um ein flächiges Schnittbild zu erzeugen, wird eine kontinuierliche Folge von Ultraschallimpulsen über einen Abtast- bzw. Scanvorgang, der elektronisch oder mechanisch sein kann, in das Gewebe eingekoppelt.

Die Vorteile des Ultraschall-Echoimpuls-Verfahrens gegenüber Röntgenbildgebungsverfahren liegen vor allem in der Gewebeschonung und in der kostengünstigen Realisierung. Ein weiterer Vorteil des Ultraschall-Echoimpuls-Verfahrens liegt in der relativ hohen Eindringtiefe des Ultraschalls in das Gewebe.

Ein Nachteil des Ultraschall-Echoimpuls-Verfahrens besteht jedoch in der vergleichsweise geringen axialen Auflösung des Ultraschallbildes. Unter der axialen Auflösung ist dabei die Auflösung entlang der Einstrahlrichtung zu verstehen. Die Auflösung entlang der Einstrahlrichtung ist von der Frequenz und der Ausdehnung des eingekoppelten Ultraschallimpulses abhängig.

Derzeit werden für den Abdominalbereich Standardfrequenzen im Bereich zwischen 5 bis 10 MHz verwendet. Bei speziellen oberflächennahen Gewebestrukturen werden heute bereits Frequenzen bis zu 50 MHz verwendet. Mit solch hohen Frequenzen wird zwar eine bessere axiale Auflösung erreicht, jedoch nimmt auch der Dämpfungskoeffizient des Gewebes mit der Frequenz linear zu, so daß bei sehr hohen Frequenzen, die im Prinzip eine höhere Auflösung ermöglichen, die Eindringtiefe des Ultraschalls in das Gewebe physikalisch bedingt stark begrenzt ist, so daß der Vorteil des Ultraschalls, gewebeschonend Tiefeninformation von dem Gewebe zu erhalten, verloren geht.

Die höchste axiale Auflösung bei der Verwendung von Hochfrequenz-Ultraschall liegt derzeit im Bereich von bis zu 30 *µ*m herab.

In der WO 97/32182 ist ein optisches bildgebendes Verfahren beschrieben, das unter der Bezeichnung "Optische Kohärenz-Tomographie" (OCT) bekannt ist. Bei der OCT wird ein Lichtstrahl erzeugt und in einen Meßlichtstrahl und einen Referenzlichtstrahl aufgespalten, wobei der Meßlichtstrahl in das zu untersuchende Gewebe eingekoppelt wird. Dabei wird der relative optische Weg zwischen dem Referenzlichtstrahl und dem Meßlichtstrahl verstellt, und es wird der vom Gewebe rückgestreute Meßlichtstrahl mit- dem Referenzlichtstrahl zu Interferenz gebracht.

In der WO 97/32182 wird in einem Ausführungsbeispiel ein Applikator zum Einkoppeln des Meßlichtstrahls in das zu untersuchende Gewebe beschrieben, der in Form eines Endoskops ausgebildet ist, in dessen Spitze ein Prisma oder ein silberbeschichteter Spiegel angeordnet ist, über den der Meßlichtstrahl senkrecht zur Längsachse des Endoskops in das zu untersuchende Gewebe eingestrahlt wird. In der Spitze des Endoskops ist ein Ultraschallwandler angeordnet, der Ultraschallwellen auf den silberbeschichteten Spiegel richtet, die dann vom dem Spiegel in entgegengesetzter Richtung zum Meßlichtstrahl in das zu untersuchende Gewebe, beispielsweise eines Hohlorgans, eingestrahlt werden.

Des weiteren ist aus der WO 98/55025 ein Ultraschall-Bildgebungsverfahren bekannt, wobei vorgeschlagen wird, das Ultraschall-Bildgebungsverfahren auch mit der optischen Kohärenz-Tomographie zu kombinieren. Dort ist allerdings nicht beschrieben, wie das Verfahren der optischen Kohärenz-Tomographie im Zusammenhang mit dem Ultraschall-Bildgebungsverfahren durchgeführt wird.

Der Erfindung liegt daher die Aufgabe zugrunde, ein bildgebendes Verfahren sowie ein bildgebendes System zu schaffen, mit denen eine höhere Auflösung des Bildgebungsverfahrens erreicht wird, um so genauere Informationen über das Gewebe zu gewinnen.

Erfindungsgemäß wird diese Aufgabe durch ein bildgebendes Verfahren zum Ermitteln eines physikalischen oder chemischen Zustands von Gewebe im menschlichen oder tierischen Körper unter Verwendung von Ultraschall gelöst, bei dem zumindest ein Ultraschallimpuls im diagnostischen Frequenz- und Leistungsbereich in das Gewebe eingekoppelt und der vom Gewebe reflektierte Ultraschallechoimpuls empfangen und in Ultraschallbildverarbeitungsmitteln verarbeitet wird, wobei zumindest ein Lichtstrahl erzeugt und in zumindest einen Meßlichtstrahl und zumindest einen Referenzlichtstrahl aufgespalten wird, der Meßlichtstrahl entlang einer gleichen Strahlachse wie der Ultraschallimpuls in das Gewebe eingekoppelt wird, und zwar so, daß der Meßlichtstrahl und der zumindest eine Ultraschallimpuls einander überlagert entlang der Strahlachse in das Gewebe eingekoppelt werden, der relative optische Weg zwischen dem Referenzlichtstrahl und dem Meßlichtstrahl verstellt wird, und der vom Gewebe rückgestreute Meßlichtstrahl mit dem Referenzlichtstrahl zu Intereferenz gebracht und in optischen Bildverarbeitungsmitteln verarbeitet wird.

Des weiteren wird diese Aufgabe erfindungsgemäß durch ein bildgebendes System zum Ermitteln eines physikalischen oder chemischen Zustands von Gewebe im menschlichen oder tierischen Körper unter Verwendung von Ultraschall gelöst, mit Ultraschallerzeugungsmitteln zum Erzeugen zumindest eines Ultraschallimpulses im diagnostischen Frequenz- und Leistungsbereich, mit Ultraschallapplikationsmitteln zum Applizieren des Ultraschallimpulses in das Gewebe, mit Ultraschallempfangsmitteln zum Empfangen des vom Gewebe reflektierten Ultraschallechoimpulses, und mit Ultraschallbildverarbeitungsmitteln zum Verarbeiten des Ultraschallechoimpulses, weiterhin mit Lichterzeugungsmitteln zum Erzeugen zumindest einen Lichtstrahles, Strahlteilermitteln zum Aufspalten des Lichtstrahles in zumindest einen Meßlichtstrahl und zumindest einen Referenzlichtstrahl, Verstellmitteln zum Verstellen des relativen optischen Weges zwischen dem Meßlichtstrahl und dem Referenzlichtstrahl, Lichtsapplikationsmitteln zum Applizieren des Meßlichtstrahles in das Gewebe, wobei die Lichtapplikationsmittel und die Ultraschallapplikationsmittel derart ausgebildet sind, daß der Ultraschallimpuls und der Meßlichtstrahl entlang einer gemeinsamen Strahlachse einander überlagert in das Gewebe einkoppelbar sind, Mitteln zum Empfangen des vom Gewebe rückgestreuten Meßlichtstrahles und Mitteln zum interferometrischen Überlagern des rückgestreuten Meßlichtstrahles mit dem Referenzlichtstrahl, sowie mit optischen Bildverarbeitungsmitteln zur Verarbeitung des interferometrischen Meßsignals.

Erfindungsgemäß wird das eingangs genannte bildgebende Verfahren unter Verwendung von Ultraschall mit dem an sich bekannten Verfahren der optischen Kohärenz-Tomographie zu einem akustisch-optischen Bildgebungsverfahren kombiniert. Dazu wird erfindungsgemäß der zumindest eine Meßlichtstrahl entlang der gleichen Strahlachse wie der Ultraschallimpuls einander überlagert in das Gewebe eingekoppelt. Bei dem bildgebenden System sind dazu die Ultraschallapplikationsmittel und die Lichtapplikationsmittel entsprechend ausgebildet, um den Ultraschall und das Licht entlang der gleichen Strahlachse in das Gewebe einzukoppeln.

Wenn sich die Verstellung des relativen optischen Weges zwischen dem Meßlichtstrahl und dem Referenzlichtstrahl auf eine Kohärenzlänge beschränkt, wird ein einziger Bildpunkt erzeugt. Eine Verstellung des optischen Weges über einen größeren Bereich als eine Kohärenzlänge erzeugt ein zunächst eindimensionales Bild in Richtung der Einstrahlachse, da das Interferenzsignal nur aus der Nähe desjenigen Objektpunkts stammt, in dem Weglängengleichheit zwischen dem Meßlichtstrahl und dem Referenzlichtstrahl besteht. Durch Verstellen des relativen optischen Wegs zwischen dem Meßlichtstrahl und dem Referenzlichtstrahl wird somit axial ein bestimmter Weg- bzw. Tiefenbereich des Gewebes in der Art eines Scannens abgetastet.

Unter "bildgebend" im Sinne der Erfindung ist im Hinblick auf den optischen Teil des Meßverfahrens demnach auch die Erzeugung eines Bildes zu verstehen, das durch eine eindimensionale Abfolge von einzelnen Bildpunkten gebildet wird. Ein solches erzeugtes Bild kann aber auch aus einem einzigen Bildpunkt bestehen. Auch die genannten Bildverarbeitungsmittel sind so zu verstehen, daß diese in der Lage sind, ein einzelnes Meßsignal zu einem einzelnen Bildpunkt zu verarbeiten.

Die Vorteile des erfindungsgemäßen Verfahrens, die sich aus der Kombination des Ultraschall-Echoimpuls-Verfahrens mit dem Verfahren der optischen Kohärenz-Tomographie ergeben, bestehen nun darin, daß mit der optischen Kohärenz-Tomographie eine axiale Auflösung erreicht wird, die höher ist als diejenige des Ultraschall-Echoimpuls-Verfahrens. Die mit der optischen Kohärenz-Tomographie erreichbare Auflösung liegt derzeit etwa im Bereich zwischen 5 bis 10 *µ*m. Die Eindringtiefe des Meßlichtes in das Gewebe ist jedoch geringer als die Eindrihgtiefe des Ultraschalls. Mit dem erfindungsgemäßen Verfahren ist es somit möglich, aus dem optisch erhaltenen Bild mit hoher Auflösung Gewebsinformationen aus oberflächennahen Bereichen des Gewebes zu gewinnen, während aus dem Ultraschallbild zusätzlich Gewebsinformationen aus tiefer liegenden Gewebsbereichen gewonnen werden können. Insbesondere im axialen Überlappungsbereich des Ultraschallbildes und des optischen Bildes lassen sich Gewebsinformationen erhalten, die zur Gewebecharakterisierung herangezogen werden können, die mit dem Ultraschall-Echoimpuls-Verfahren oder dem Verfahren der optischen Kohärenz-Tomographie allein nicht gewonnen werden können. So kann beispielsweise das optische Bild zur Ermittlung der Dicke einer Gewebsschicht herangezogen werden, wobei dann aus der optisch ermittelten Dicke der Gewebsschicht aus dem zeitlichen Abstand zweier Ultraschallechoimpulse die Ultraschallgeschwindigkeit und aus dieser die Elastizität und Dichte des Gewebes ermittelt werden können. Allgemein kann mit dem Ultraschallbild sowohl über die Gewebeoberfläche als auch in die Tiefe eine Übersichtsdarstellung gewonnen werden. Dabei werden verdächtige Areale identifiziert, die mittels der optischen Kohärenztomographie detailliert betrachtet werden können. Die optische Kohärenztomographie hat in Verbindung mit dem Ultraschallverfahren mit anderen Worten die Funktion eines Zoom.

Das Verfahren kann darüber hinaus zur Therapiesteuerung verwendet werden. So verstärkt sich bspw. beim Skin-Resurfacing durch die Koagulation das optische Interferenzmeßsignal und die Therapie kann im geeigneten Moment gestoppt werden.

Mit dem erfindungsgemäßen Verfahren bzw. dem erfindungsgemäßen System wird eine Analysemethode geschaffen, die eine Gewebedifferenzierung und die Ermittlung pathologischer Veränderungen in der Oberflächenstruktur von Gewebe ermöglicht. Darüber hinaus können dynamische Prozesse, wie bspw. der Blutfluß oder Bewegungsabläufe im Gewebe sichtbar gemacht werden, indem das Verfahren bspw. im Dopplermodus durchgeführt oder eine schnelle Abfolge von Einzelbildern erzeugt wird. Schließlich wird eine funktionelle Bildgebung ermöglicht. Mögliche Anwendungen sind beispielsweise die endoskopische Quantifizierung des Knorpelgewebes in Gelenken oder die endoskopische Quantifizierung der epithelialen Strukturen von Hohlorganen, Hautstrukturuntersuchungen, usw.

Somit wird die der Erfindung zugrunde liegende Aufgabe vollkommen gelöst.

In einer bevorzugten Ausgestaltung des Verfahrens wird der Lichtstrahl mit einer spektralen Bandbreite im Bereich zwischen 10 und 200 nm erzeugt.

Bei dem erfindungsgemäßen System weisen die Lichterzeugungsmittel dazu eine Lichtquelle mit einer spektralen Bandbreite im Bereich zwischen 10 und 200 nm auf.

Die axiale Auflösung des aus der optischen Kohärenz-Tomographie gewonnenen Bildes erhöht sich mit der Zunahme der spektralen Bandbreite bzw. mit der Abnahme der Kohärenzlänge des verwendeten Lichtes. Durch die Verwendung einer Lichtquelle mit einer hohen spektralen Bandbreite wird somit vorteilhaft eine hohe Auflösung des optisch erhaltenen Bildes erzielt und ermöglicht beispielsweise eine sehr genaue Messung eines ortsabhängigen Parameters bzw. Ermittlung des Gewebszustands. Als Lichtquelle kann beispielsweise eine Superlumineszenzdiode mit einer spektralen Bandbreite von 30 nm und einer Leistung von 1,5 mW verwendet werden.

In einer weiteren bevorzugten Ausgestaltung des Verfahrens wird der relative optische Weg zwischen dem Referenzlichtstrahl und dem Meßlichtstrahl über eine Kohärenzlänge des Lichts hinaus verstellt.

Die Verstellung des relativen optischen Wegs zwischen dem Referenzlichtstrahl und dem Meßlichtstrahl über eine Kohärenzlänge hinaus erzeugt vorteilhafterweise zumindest ein eindimensionales optisches (Tiefen-)Bild in Einstrahlrichtung, d.h. durch diese Maßnahme wird das Gewebe in Einstrahlrichtung optisch abgetastet.

In einer weiteren bevorzugten Ausgestaltung des Verfahrens wird eine kontinuierliche Folge von Ultraschallimpulsen als Ultraschallstrahl in das Gewebe eingekoppelt, wobei der Ultraschallstrahl und der Meßlichtstrahl entlang der Strahlachse einander überlagert sind.

Mit dieser Maßnahme ist es möglich, nicht nur eindimensionale, sondern auch zweidimensionale Schnittbilder zu erhalten, indem beispielsweise der Ultraschallstrahl und der Meßlichtstrahl gemeinsam verfahren werden, oder durch flächig ausgebildete Applikationsmittel in das Gewebe eingekoppelt werden. An dieser Stelle sei erwähnt, daß der Lichtstrahl und damit der Meßlichtstrahl ebenfalls aus einer Folge von Lichtimpulsen bestehen oder kontinuierlich sein kann.

Dabei ist es gemäß dem Verfahren bevorzugt, wenn die gemeinsame Strahlachse des Ultraschallstrahls und des Meßlichtstrahls in einer Ebene parallel zur Oberfläche des Gewebes verfahren wird.

Bei dem System sind dazu weiterhin Mittel zum Verfahren der Strahlachse in einer Ebene parallel zur Oberfläche des Gewebes vorgesehen.

Durch diese Maßnahmen kann das Gewebe mit geringem technischem Aufwand lateral mit dem Ultraschall und dem Licht abgetastet werden, um ein zweidimensionales Ultraschall-/optisches Schnittbild zu erhalten. Andere bevorzugte Möglichkeiten, ein zweidimensionales Schnittbild zu erzeugen, bestehen in der Verwendung flächig abbildender Ultraschall- und optischer Systeme, bspw. Arrays.

Weiterhin ist es bevorzugt, wenn die gemeinsame Strahlachse des Ultraschallstrahls und des Lichtstrahls um eine Drehachse quer zur momentanen Einstrahlrichtung geschwenkt wird.

Bei dem System sind dazu entsprechend Mittel zum Drehen der Strahlachse um eine Drehachse quer zur momentanen Einstrahlrichtung vorgesehen.

Bei dieser Ausgestaltung des Verfahrens und des Systems eignen sich diese insbesondere zur Erzeugung von Ultraschall/optischen Schnittbildern eines Hohlorgans.

In einer weiteren bevorzugten Ausgestaltung des Verfahrens werden das durch die Verarbeitung des rückgestreuten Meßlichtstrahls optisch erhaltene Bild und das durch Verarbeitung des Ultraschallechoimpulses erhaltene Ultraschallbild miteinander derart kombiniert, daß im Nahbereich das optisch erhaltene Bild und im Fernbereich das Ultraschallbild dargestellt werden.

Bei dem System sind dazu die Ultraschallbildverarbeitungsmittel und die optischen Bildverarbeitungsmittel miteinander derart gekoppelt, daß das Ultraschallbild und das optisch gewonnene Bild einander überlagert darstellbar sind.

Hierbei ist von Vorteil, daß im Nahbereich sehr hoch aufgelöst werden kann, bzw. im Fernbereich zusätzliche Informationen über das Gewebe durch das Ultraschallbild zur Verfügung steht. Bei hochfrequenten isolierten Ultraschallbildern ist meist die Gewebeoberfläche nicht zu differenzieren, da starke Oberflächenechos am Gewebeübergang Echos aus geringfügig tiefer liegenden Strukturen überlagern. Durch die erfindungsgemäße Kombination mit der optischen Kohärenz-Tomographie wird nun eine hoch auflösende Differenzierung der Gewebeoberfläche ermöglicht.

In einer weiteren bevorzugten Ausgestaltung des Verfahrens wird mittels des durch die Verarbeitung des rückgestreuten Meßlichtstrahls optisch erhaltenen Bildes die Dicke einer oberflächennahen Gewebeschicht, aus dem Ultraschallbild die Laufzeitdifferenz zwischen dem an einer ersten Gewebeschichtsgrenze reflektierten Ultraschallechoimpuls und dem an einer zweiten Gewebeschichtsgrenze reflektierten Ultraschallechomimpuls, und aus der Laufzeitdifferenz und der Dicke die Schallausbreitungsgeschwindigkeit in der Gewebeschicht ermittelt.

Wie bereits erwähnt, eröffnet die erfindungsgemäße Kombination des Ultraschall-Echoimpuls-Verfahrens mit der optischen Kohärenz-Tomographie neue Möglichkeiten, Zustände des untersuchten Gewebes zu ermitteln, die mit einem Ultraschall- oder optischen Bildgebungsverfahren allein bislang nicht ermittelt werden konnten. Durch die zuvor beschriebene Maßnahme ist es möglich, aus der so ermittelten Schallausbreitungsgeschwindigkeit die Elastizität und Dichte des untersuchten Gewebes zu ermitteln.

Bevorzugte Anwendungen des erfindungsgemäßen Verfahrens und/oder der erfindungsgemäßen Vorrichtung sind, wie bereits erwähnt, die Gewebedifferenzierung und/oder die Ermittlung pathologischer Veränderungen in der Oberflächenstruktur von Gewebe.

Bei einer bevorzugten Ausgestaltung wird das Ultraschallbild als Übersichtsdarstellung des untersuchten Gewebes und das optisch erhaltene Bild für eine Detaildarstellung ausgewählter Gewebebereiche herangezogen.

Das eine höhere Eindringtiefe ermöglichende Ultraschallbildverfahren und die eine höhere Auflösung bietende optische Kohärenztomograhie können durch eine entsprechende Bildverarbeitung, insbesondere das Merging-Verfahren, so miteinander gekoppelt werden, daß aus dem optisch erhaltenen Bild Details des untersuchten Gewebes erkennbar werden, wobei zuvor aus dem Ultraschallbild in einer "Grobübersicht" Gewebebereiche identifiziert werden, die möglicherweise pathologisch verändert sind und einer näheren Untersuchung bedürfen.

Das Verfahren und die Vorrichtung können darüber hinaus für die Therapiesteuerung verwendet werden, wie bereits vorstehend erwähnt wurde. Bei einer weiteren bevorzugten Ausgestaltung des Verfahrens wird zusätzlich durch den Meßlichtstrahl oder durch unabhängig von diesem in das Gewebe eingestrahltes Licht eine Fluoreszenz im Gewebe angeregt, und wird das Fluoreszenzlicht empfangen und das Fluoreszenzlichtbild zusätzlich zu dem optisch erhaltenen Bild dargestellt.

Bei dieser Ausgestaltung des erfindungsgemäßen Verfahrens wird dieses mit der sogenannten photodynamischen Diagnose (PDD) kombiniert. Bei der photodynamischen Diagnose wird gegebenenfalls nach Verabreichung einer lichtsensitiven Substanz in das Gewebe eine Fluoreszenz angeregt, die zur weiteren Differenzierung, insbesondere zur Differenzierung von gesundem Gewebe von pathologisch verändertem Gewebe herangezogen werden kann. Hierbei kann sowohl eine körpereigene Autofluoreszenz oder auch durch, wie vorstehend erwähnt, verabreichte Pharmaka induzierte Xenofluoreszenz zur Erzeugung von Oberflächen- oder Schnittbildern herangezogen werden. Diese können sowohl zur Identifikation verdächtiger Areale verwendet werden, als auch zur Unterstützung bzw. Ergänzung der durch das Ultraschallbild und das optisch erhaltene Bild gewonnenen Aussage zur weiteren Differenzierung herangezogen werden.

Auch ohne das Ultraschallbildverfahren kann die optische Kohärenztomograpie mit der photodynamischen Diagnose kombiniert werden, auch wenn in der vorliegenden Beschreibung vorteilhaft die Kombination aus Ultraschallbild, optischer Kohärenztomographie und photodynamischer Diagnose beschrieben ist.

Bei einer weiteren bevorzugten Ausgestaltung des Systems sind die Ultraschallapplikationsmittel und die Lichtapplikationsmittel zusammen in einem als Endoskop ausgebildeten Applikator integriert.

Ein endoskopischer Applikator eignet sich insbesondere zur endoskopischen Quantifizierung des Knorpelgewebes bspw. in Gelenken oder der endoskopischen Quantifizierung der epithelialen Strukturen von Hohlorganen.

Dabei ist es weiterhin bevorzugt, wenn die Ultraschallerzeugungsmittel zumindest einen piezoelektrischen Ultraschallwandler und die Lichtapplikationsmittel zumindest einen etwa mittig in einer Abstrahlfläche des Ultraschallwandlers endenden Lichtleiter aufweisen.

Die Realisierung des Systems mit einem endoskopischen Applikator hat den Vorteil, daß nur ein Applikationssystem eingesetzt werden muß und keine zusätzlichen Ablenksysteme benötigt werden, um den Ultraschallstrahl und den Lichtstrahl entlang der gleichen Strahlachse in das Gewebe einzukoppeln.

In einer weiteren bevorzugten Ausgestaltung weisen die Lichtapplikationsmittel und die Ultraschallapplikationsmittel eine Spiegelanordnung auf, die für Ultraschall durchlässig und fär Licht reflektierend, oder umgekehrt ist, um den Ultraschallimpuls und den Meßlichtstrahl entlang der gemeinsamen Strahlachse einzukoppeln.

Hierbei ist von Vorteil, daß bereits vorhandene Applikatoren, nämlich einerseits ein separater Ultraschallapplikator und andererseits ein separater Lichtapplikator verwendet werden können, wobei dann mittels der Spiegelanordnung der Ultraschallstrahl und der Lichtstrahl so einander überlagert werden können, daß beide entlang der gleichen Strahlachse in das Gewebe eingekoppelt werden.

In einer weiteren bevorzugten Ausgestaltung weisen die Strahlteilermittel und die Mittel zum interferometrischen Überlagern des rückgestreuten Meßlichtstrahls mit dem Referenzlichtstrahl ein Zwei- oder Mehrstrahlinterferometer, bevorzugt ein Michelson-Interferometer, auf.

Der Einsatz eines Zwei- oder Mehrstrahlinterferometers, bevorzugt eines Michelson-Interferometers hat sich bislang in der optischen Kohärenz-Tomographie bewährt und läßt sich ebenso besonders kostengünstig in das erfindungsgemäße System implementieren, beispielsweise in einer optischen Ansteuerungs- und Auswerteeinheit.

Weitere Vorteile ergeben sich aus der nachfolgenden Beschreibung und der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden hiernach mit Bezug auf diese näher beschrieben. Es zeigen:
- Fig. 1: ein erfindungsgemäßes bildgebendes System zum Ermitteln eines physikalischen oder chemischen Zustands von Gewebe im menschlichen oder tierischen Körper in einer äußerst schematischen Gesamtdarstellung, teilweise im Längsschnitt;
- Fig. 2: eine schematische Darstellung eines Teiles des Systems in Fig. 1;
- Fig. 3: ein Schema zur Veranschaulichung der Ermittlung eines beispielhaften ortsabhängigen Parameters des Gewebes;
- Fig. 4: ein weiteres Ausführungsbeispiel eines bildgebenden Systems im Bereich des Applikationssystems in einer schematischen Darstellung im Längsschnitt;
- Fig. 5: ein weiteres Ausführungsbeispiel eines bildgebenden Systems im Bereich des Applikationssystems in einer schematischen Darstellung im Längsschnitt;
- Fig. 6: ein weiteres Ausführungsbeispiel eines bildgebenden Systems im Bereich des Applikationssystems in einer schematischen Darstellung im Längsschnitt; und
- Fig. 7: ein noch weiteres Ausführungsbeispiel eines bildgebenden Systems im Bereich des Applikationssystems in einer schematischen Darstellung im Längsschnitt.

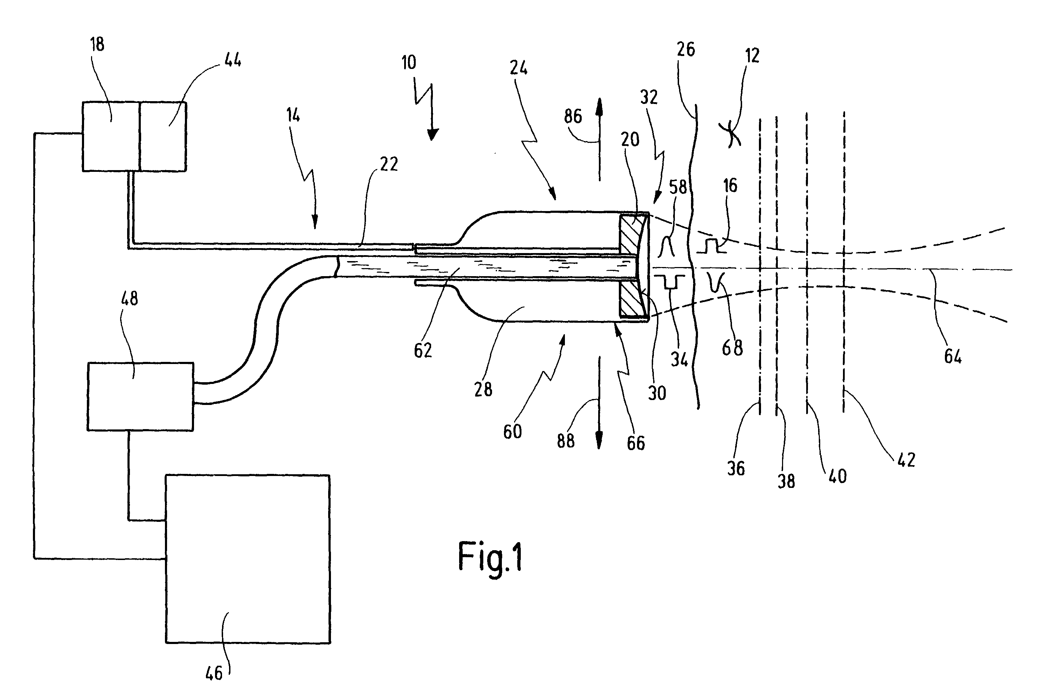

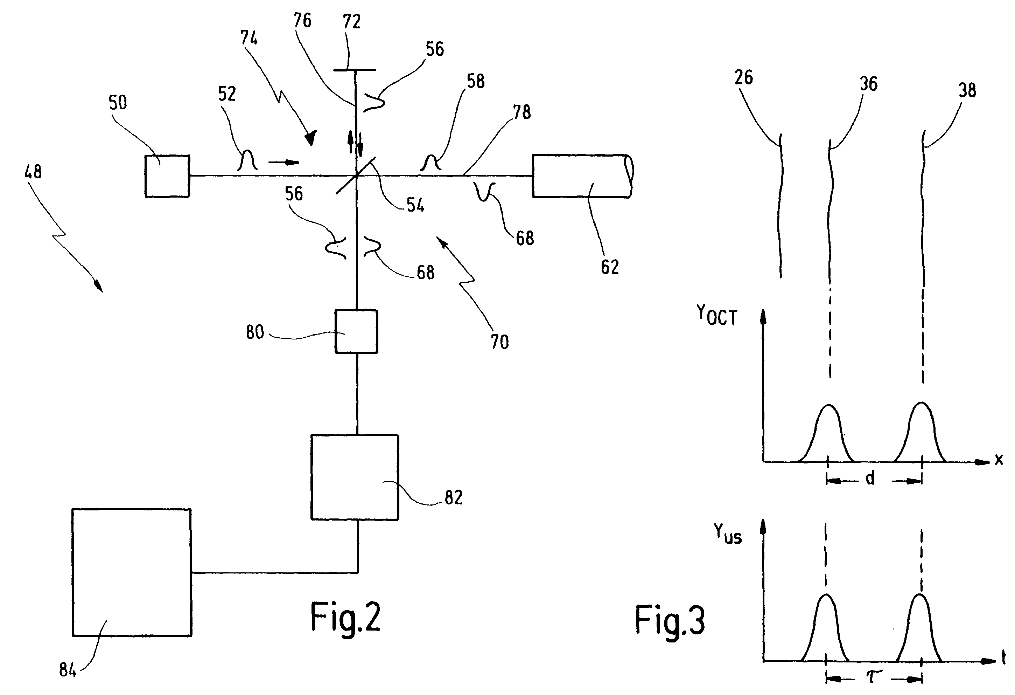

In Fig. 1 und 2 ist ein mit dem allgemeinen Bezugszeichen 10 versehenes bildgebendes System zum Ermitteln eines physikalischen oder chemischen Zustands von Gewebe 12 im menschlichen oder tierischen Körper unter Verwendung von Ultraschall dargestellt.

Mit dem bildgebenden System 10 wird ein bildgebendes Verfahren durchgeführt, das der Gewebedifferenzierung bzw. Erfassung pathologischer Veränderungen des Gewebes 12 durch die Ermittlung eines physikalischen oder chemischen Zustands, beispielsweise eines oder mehrerer ortsabhängiger Parameter des Gewebes, dient, wobei darunter auch einfach die sichtbare Darstellung des Gewebes in einer Darstellungseinheit zu verstehen ist.

Das Gewebe 12 ist beispielsweise Hautgewebe, Knorpelgewebe oder dergleichen.

Das System 10 weist zunächst Ultraschallerzeugungsmittel 14 zum Erzeugen zumindest eines Ultraschallimpulses 16 auf.

Die Ultraschallerzeugungsmittel 14 umfassen einen Impulsgenerator 18, der zumindest einen elektrischen Impuls erzeugt. Die Ultraschallerzeugungsmittel 14 umfassen ferner einen piezoelektrischen Ultraschallwandler 20. Der Ultraschallwandler 20 ist mit dem Impulsgenerator 18 über eine elektrische Leitung 22 verbunden.

Der vom Impulsgenerator 18 erzeugte elektrische Impuls wird über die Leitung 22 dem Ultraschallwandler 20 zugeführt, der den elektrischen Impuls in den Ultraschallimpuls 16 umwandelt.

Üblicherweise wird eine kontinuierliche Folge von elektrischen Impulsen im Impulsgenerator 18 und dementsprechend eine kontinuierliche Folge von Ultraschallimpulsen 16 durch den Ultraschallwandler 20 erzeugt.

Die von den Ultraschallerzeugungsmitteln 14, genauer gesagt vom Ultraschallwandler 20 erzeugten Ultraschallimpulse 16 weisen eine Frequenz und Leistung im diagnostischen Bereich auf. Ultraschallfrequenzen im diagnostischen Bereich liegen etwa im Bereich zwischen 10 und 50 MHz. Die Leistung der Ultraschallimpulse 16 ist so gewählt, daß das Gewebe 12 durch die eingestrahlte Ultraschallenergie nicht beeinträchtigt, d.h. geschädigt wird. Die Leistung des Ultraschalls ist möglichst so gering, daß eine Erwärmung des Gewebes 12, auf jeden Fall Kavitationseffekte, die das Gewebe 12 zerstören, vermieden werden.

Das System 10 weist weiterhin Ultraschallapplikationsmittel 24 auf, um den Ultraschallimpuls 16 bzw. die Ultraschallimpulse 16 in das Gewebe 12 durch eine Gewebsoberfläche 26 hindurch einzukoppeln. Die Ultraschallapplikationsmittel 24 sind als Applikator 28 ausgebildet, in dem der Ultraschallwandler 20 distalseitig angeordnet ist.

Der Applikator 28 ist als Endoskop ausgebildet, so daß mittels des Applikators 28 der Ultraschallwandler 20 nahe an die Gewebsoberfläche 26 des zu untersuchenden Gewebes 12 herangebracht werden kann, auch wenn es sich bei dem Gewebe 12 um Gewebe innerhalb des menschlichen Körpers, beispielsweise eines Organs, handelt. Der Applikator 28 eignet sich jedoch auch zur extrakorporalen Applikation von Ultraschall in Hautstrukturen.

In Fig. 1 ist eine Abstrahlfläche 30 des Ultraschallwandlers 20 von der Gewebsoberfläche 26 beabstandet dargestellt. Da eine Fortpflanzung von Hochfrequenz im Ultraschall über Luft beinah unmöglich ist, muß zwischen die Abstrahlfläche 30 und die Gewebsoberfläche 26 dann, wenn die Abstrahlfläche 30 nicht dicht an die Gewebsoberfläche 26 herangeführt werden kann, ein Koppelmedium, beispielsweise in Form eines Gels oder eines Festkörpers, gebracht werden.

Das System 10 weist weiterhin Ultraschallempfangsmittel 32 zum Empfangen des im Gewebe 12 reflektierten Ultraschallechoimpulses auf.

Beim Einkoppeln des Ultraschallimpulses 16 wird dieser an der Gewebsoberfläche 26 sowie an weiteren Gewebsgrenzschichten, die beispielhaft dargestellt und mit 36, 38, 40 und 42 bezeichnet sind, teilweise reflektiert und teilweise durchgelassen, so daß von jeder dieser Gewebsgrenzschichten 36, 38, 40 und 42 jeweils ein Ultraschallechoimpuls 34 zurück reflektiert wird. Die Ultraschallempfangsmittel 32 werden in dem gezeigten Ausführungsbeispiel durch denselben Ultraschallwandler 20 gebildet, der auch die Ultraschallimpulse 16 erzeugt, wobei jedoch auch separate piezoelektrische Empfänger oder ein Wellenleiter vorgesehen sein können. Ebenso kann anstelle des Ultraschallwandlers 20 auch ein Wellenleiter vorgesehen sein, durch den extern erzeugter Ultraschall in den Applikator 28 zugeführt und von diesem in das Gewebe 12 appliziert wird.

Der Ultraschallwandler 20 wandelt den bzw. die empfangenen Ultraschallechoimpulse in elektrische Impulse um, die in den Impulsgenerator 18 weitergeleitet werden.

Der Impulsgenerator 18 ist mit Ultraschallbildverarbeitungsmitteln 44 ausgestattet, die die empfangenen Ultraschallechoimpulse 34 quantitativ auswerten.

Die so ausgewerteten Ultraschallechoimpulse werden anschließend weiterhin einer Darstellungseinheit 46 zur sichtbaren Darstellung und ggfls. weiteren Auswertung des Ultraschallbildes zugeführt.

Die zuvor beschriebenen, das Ultraschallbildgebungsverfahren betreffenden Komponenten des Systems 10 sind nun erfindungsgemäß mit hiernach beschriebenen Komponenten kombiniert, mit denen in Kombination mit dem Ultraschallbildgebungsverfahren ein optisches Bildgebungsverfahren, genauer gesagt die optische Kohärenz-Tomographie, durchgeführt wird.

Zu den Grundlagen der optischen Kohärenz-Tomographie sowie zur Erläuterung hier nicht näher erläuterter Begriffe wird auf den Übersichtsartikel von Adolf F. Fercher, "Optical Coherence Tomography" in: Journal of Biomedical Optics, Band 1, Nr. 2, April 1996, verwiesen.

Das System 10 weist dazu eine Ansteuerungs- und Verarbeitungseinheit 48 für die optische Kohärenz-Tomographie auf.

Die Ansteuerungs- und Verarbeitungseinheit 48 ist in Fig. 2 näher dargestellt.

Die Ansteuerungs- und Verarbeitungseinheit 48 weist zunächst Lichterzeugungsmittel 50 auf. Die Lichterzeugungsmittel 50 weisen eine Lichtquelle mit einer spektralen Bandbreite im Bereich zwischen 10 und 200 nm auf. Eine Lichtquelle mit solchen spektralen Eigenschaften wird beispielsweise durch eine Superlumineszenzdiode mit einer spektralen Bandbreite von 30 nm und einer Leistung von 1,5 mW bereitgestellt.

Die Lichterzeugungsmittel 50 erzeugen zumindest einen Lichtstrahl 52, der hier durch eine kontinuierliche Folge von Lichtimpulsen gebildet wird, wobei aus Gründen der vereinfachten Darstellung der Lichtstrahl 52 repräsentativ durch einen einzelnen Impuls dargestellt ist.

Weiterhin sind Strahlteilermittel 54 vorgesehen, mit dem der erzeugte Lichtstrahl 52 in einen Referenzlichtstrahl 56 und einen Meßlichtstrahl 58 aufgespalten wird.

Das System 10 weist weiterhin gemäß Fig. 1 Lichtapplikationsmittel 60 zum Einkoppeln des Meßlichtstrahls 58 in das Gewebe 12 auf. Die Lichtapplikationsmittel 60 sind ebenfalls im Applikator 28 integriert und weisen einen Lichtleiter 62 auf, der eine einzelne Lichtleitfaser oder ein Faserbündel aufweist.

Die Lichtapplikationsmittel 60 und die Ultraschallapplikationsmittel 24 sind nun so ausgebildet, daß die Ultraschallimpulse 16 und der Meßlichtstrahl 58 entlang einer gleichen Strahlachse 64 in das Gewebe 12 eingekoppelt werden. Dies ermöglicht es, einen selben Gewebebereich des Gewebes 12 sowohl mit Ultraschall als auch mit Licht zu bestrahlen, so daß über diesen gemeinsam mit Ultraschall und Licht bestrahlten Gewebebereich Informationen aus dem Ultraschallbild und aus dem mittels der optischen Kohärenz-Tomographie gewonnenen optischen Bild gewonnen werden können.

Bei diesem Ausführungsbeispiel wird das Einkoppeln der Ultraschallimpulse 16 und des Meßlichtstrahls 58 entlang der gemeinsamen Strahlachse 64 dadurch erreicht, daß der Lichtleiter 62 ebenfalls in dem als Endoskop ausgebildeten Applikator 28 positioniert ist und etwa mittig in der Abstrahlfläche 30 des Ultraschallwandlers 20 endet.

Der Lichtleiter 62 bildet außerdem Lichtempfangsmittel 66 zum Empfangen des vom Gewebe 12, beispielsweise an den Gewebsgrenzschichten 36, 38 und/oder 40 rückgestreuten Meßlichtstrahls 68.

Der rückgestreute Meßlichtstrahl 68 wird über den Lichtleiter 62 in die Ansteuerungs- und Verarbeitungseinheit 48 zurückgeführt. Die Ansteuerungs- und Verarbeitungseinheit 48 weist weiterhin Mittel 70 zum interferometrischen Überlagern des rückgestreuten Meßlichtstrahls 68 mit dem Referenzlichtstrahl 56 auf, um beide Strahlen miteinander zur Interferenz zu bringen. Die Mittel 70 zum interferometrischen Überlagern des rückgestreuten Meßlichtimpulses 68 und des Referenzlichtimpulses 56 werden einerseits durch die Strahlteilermittel 54 und andererseits durch einen Spiegel 72 gebildet.

Die Strahlteilermittel 54 und die Mittel 70 zum interferometrischen Überlagern des rückgestreuten Meßlichtstrahls 68 mit dem Referenzlichtstrahl 56 werden in dem gezeigten Ausführungsbeispiel durch ein Zweitstrahlinterferometer 74, das als Michelson-Interferometer ausgebildet ist, gebildet. Das Michelson-Interferometer ist, genauer gesagt, ein teilweises Michelson-Interferometer, denn der üblicherweise für ein vollständiges Michelson-Interferometer vorgesehene Spiegel im Meßarm ist durch das zu untersuchende Gewebe ersetzt.

Die Strahlteilermittel 54 werden dabei durch eine planparallele halbverspiegelte Glasplatte gebildet, die schräg zur Einstrahlrichtung des Lichtstrahls 52 steht. Das Zweistrahlinterferometer 74 weist demnach einen Referenzarm 76 und einen Meßarm 78 auf. Der Referenzarm 76 kann dabei verstellt werden, um die optische Weglänge des Referenzarmes 76 in Bezug zur optischen Weglänge des Meßarms 78 zu verstellen. Es ist jedoch auch möglich, den Meßarm 78 verstellbar zu gestalten und diesen zu verstellen, um den relativen optischen Weg zwischen dem Referenzarm 76 und dem Meßarm 78 zu variieren. Andere Möglichkeiten bestehen beispielsweise in einer Bewegung des Objekts, hier des Gewebes relativ zu dem Meßsystem.

Realisierungen für die Verstellbarkeit des optischen Wegs über die Verstellung des optischen Wegs des Referenzarms 76 bestehen bspw. in einer mechanischen Bewegung des Spiegels 72, in einer Beeinflussung des Lichtwegs des Referenzlichtstrahls 56 in Glasfasern über Piezoelemente oder, ohne mechanische Verstellung, durch dispersive Elemente im Lichtweg.

Die Ansteuerungs- und Verarbeitungseinheit 48 weist weiterhin einen Photodetektor 80 auf, der die Amplitude des Signals der interferometrischen Überlagerung aus dem rückgestreuten Meßlichtstrahl 68 und dem Referenzlichtstrahl 56 detektiert. Dazu enthält der Photodetektor 80 bereits elektronische Mittel, die eine Detektion der Amplitude des Interferenzsignals ermöglichen, beispielsweise durch Abzug des DC-Anteils, Gleichrichtung und Mittelung. Das von dem Photodetektor 80 erzeugte Meßsignal wird dann einem Verstärker 82 zugeführt. Das verstärkte Signal wird schließlich dann optischen Bildverarbeitungsmitteln 34 zur Verarbeitung des interferometrischen Meßsignals zugeführt.

Die optischen Bildverarbeitungsmittel 84 und die Ultraschallbildverarbeitungsmittel 44 werden dann in der Darstellungseinheit 46 miteinander kombiniert.

Das System 10 weist weiterhin nicht näher dargestellte Mittel zum Verfahren der Strahlachse 64 in einer Ebene parallel zur Gewebsoberfläche 26 auf, wie mit Pfeilen 86 und 88 angedeutet ist.

Wie bereits erwähnt, werden der oder die Ultraschallimpulse 16 und der Meßlichtstrahl 58 entlang der gemeinsamen Strahlachse 64 in das Gewebe 12 eingekoppelt. Die in das Gewebe 12 eingekoppelten Ultraschallimpulse 16 dringen tiefer in das Gewebe 12 ein als der Meßlichtstrahl 58. Andererseits läßt sich aus dem rückgestreuten Meßlichtstrahl 68 ein Bild gewinnen, das eine um etwa den Faktor 10 höhere Auflösung bietet als das durch Verarbeitung der Ultraschallechoimpulse 34 gewonnene Ultraschallbild. Durch die durch das System 10 ermöglichte Kombination des Ultraschall-Echoimpuls-Verfahrens mit der optischen Kohärenz-Tomographie lassen sich nun beide Vorteile, nämlich einerseits die höhere Eindringtiefe des Ultraschalls und andererseits die höhere Auflösung des Kohärenz-Tomographiebildes, optimal ausnutzen.

In der Darstellungseinheit 46 werden das Ultraschallbild und das optisch erhaltene Bild derart miteinander kombiniert, daß im Nahbereich das optisch erhaltene Bild und im Fernbereich das Ultraschallbild dargestellt werden. Mittels des optisch erhaltenen Bildes lassen sich beispielsweise die Gewebsschichten zwischen den Gewebsgrenzschichten 36 und 38 bzw. 38 und 40 mit hoher Auflösung darstellen, während aus dem Ultraschallbild weitere Gewebsinformation aus dem Bereich zwischen den Gewebsgrenzschichten 40 und 42 entnommen werden kann.

Aus der Kombination des Ultraschallbildes und dem durch die optische Kohärenz-Tomographie erhaltenen optischen Bildes läßt sich beispielsweise die Schallausbreitungsgeschwindigkeit der Gewebsschicht zwischen den Gewebsgrenzschichten 36 und 38 und damit die Dichte und Elastizität dieses Gewebes ermitteln.

Dazu ist in Fig. 3 im oberen Diagramm schematisch das optisch erhaltene Bild dargestellt, wobei entlang der Y-Achse die Amplitude des vom Photodetektor 80 empfangenen interferometrischen Meßsignals und entlang der X-Achse die Weglänge entlang der Strahlachse 64 abgetragen ist. Aus dem Abstand des zur Gewebsgrenzschicht 36 zugehörigen Meßsignals, das dem an dieser Schichtgrenze rückgestreuten Meßlichtstrahl 68 zugeordnet ist, und des zur Gewebsschichtgrenze 38 zugehörigen Meßsignals, das dem an dieser Schichtgrenze rückgestreuten Meßlichtstrahl 68 zugeordnet ist, läßt sich durch die optische Kohärenz-Tomographie interferometrisch die Dicke d der Gewebsschicht zwischen den beiden Gewebsgrenzschichten 36 und 38 bestimmen. Im Sinne der Erfindung stellt die Abfolge der in Fig. 3 dargestellten Meßsignale ein "Bild" dar.

Im unteren Diagramm in Fig. 3 ist das zugehörige Ultraschallbild dargestellt, wobei entlang der Y-Achse wiederum die Amplitude des Ultraschall - Meßsignals und entlang der X-Achse die Laufzeit der Ultraschall-Echoimpulse 34 abgetragen ist. Aus diesem Diagramm läßt sich die Laufzeitdifferenz τ zwischen dem an der ersten Gewebeschichtsgrenze 36 reflektierten Ultraschallechoimpuls 34 und dem an der zweiten Gewebeschichtsgrenze 38 reflektierten Ultraschallechoimpuls 34 entnehmen.

Aus der Dicke d und der Laufzeitdifferenz τ ergibt sich dann die Schallausbreitungsgeschwindigkeit c im Gewebsbereich zwischen den Gewebsgrenzschichten 36 und 38 als c = 2d/τ. Aus dieser Analyse kann dann Gewebsdifferenzierung betrieben und können pathologische Veränderungen in der Oberflächenstruktur des Gewebes 12 durch Vergleich mit entsprechenden Parametern von gesundem Gewebe detektiert werden.

Während sich das System 10 mit dem als Endoskop ausgebildeten Applikator 28 insbesondere zur eindimensionalen Bilddarstellung eignet, kann mit dem System 10 durch Verfahren der Einstrahlachse 64 parallel zur Gewebsoberfläche 26 auch ein zweidimensionales Ultraschall-/optisches Schnittbild erzeugt werden.

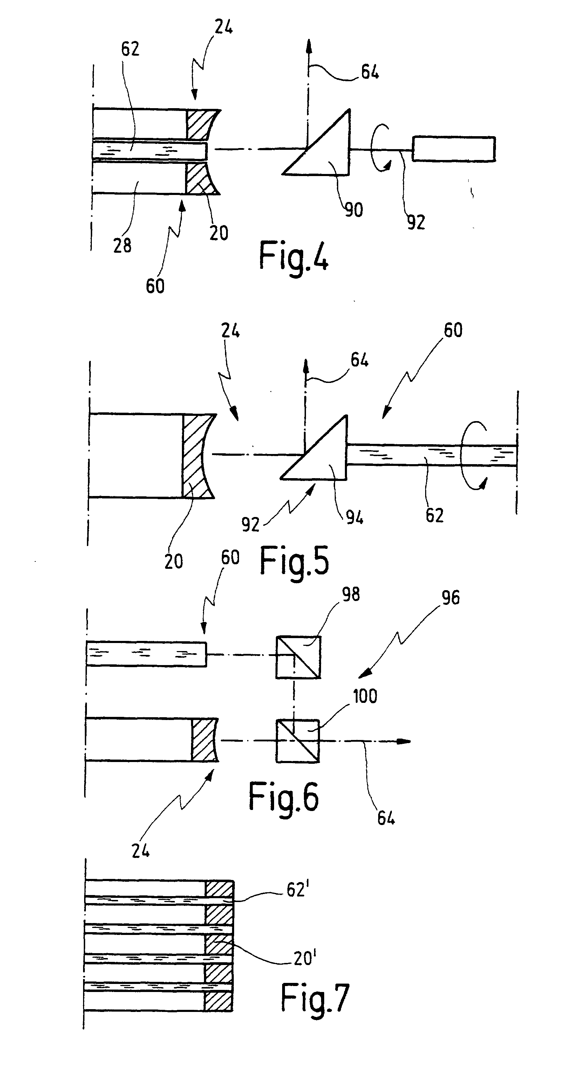

In Fig. 4 bis 7 sind weitere Ausführungsbeispiele für bildgebende Systeme gemäß der vorliegenden Erfindung dargestellt, wobei sich die Darstellung auf den Bereich der Applikationssysteme dieser bildgebenden Systeme beschränkt. Gleiche Teile sind mit den gleichen Bezugszeichen versehen wie bei dem System 10.

Bei dem in Fig. 4 dargestellten Ausführungsbeispiel umfassen die Ultraschallapplikationsmittel 24 und die Lichtapplikationsmittel 60 weiterhin einen Spiegel 90, der sowohl für Ultraschall als auch für Licht reflektierend ist. Nach Auftreffen des Ultraschalls und des Lichts auf den Spiegel 90 wird die Strahlachse 64 rechtwinklig abgelenkt. Der Spiegel 90 ist weiterhin um eine Achse 92, die mit der reflektierenden Fläche einen Winkel von etwa 45° bildet, um 360° drehbar. Durch diese Anordnung wird es ermöglicht, die gemeinsame Strahlachse 64 des Ultraschallstrahls und des Lichtstrahls um die Drehachse 92, die quer zur momentanen Einstrahlrichtung verläuft, zu verfahren. Mit diesem Applikationssystem können Querschnittsbilder von Gefäßwandstrukturen von Hohlorganen erzeugt werden.

Bei dem in Fig. 5 dargestellten Ausführungsbeispiel sind die Lichtapplikationsmittel 60 und die Ultraschallapplikationsmittel 24 nicht in einem einzigen gemeinsamen Applikator vereint, sondern getrennt ausgeführt. Die Lichtapplikationsmittel 60 und die Ultraschallapplikationsmittel 24 weisen weiterhin eine Spiegelanordnung 92 auf, die einen um 45° geneigt angeordneten Spiegel 94 aufweisen, wobei der Spiegel 94 für Licht durchlässig und für Ultraschall reflektierend ist. Durch den Spiegel 94 wird die Strahlachse 64 wiederum rechtwinklig abgelenkt. Durch Drehen des Spiegels 94 um die Achse des Lichtleiters 92 kann die Strahlachse 64 wiederum um 360° gedreht werden, um ein Querschnittsbild eines Hohlorgans zu erzeugen.

In Fig. 6 sind die Ultraschallapplikationsmittel 24 und die Lichtapplikationsmittel 60 wiederum getrennt ausgeführt. Der Ultraschallstrahl und der Meßlichtstrahl werden über eine Spiegelanordnung 96 miteinander entlang der Strahlachse 64 vereint. Die Spiegelanordnung 96 weist einen ersten Spiegel 98, der für Licht reflektierend, sowie einen zweiten Spiegel 100 auf, der für Licht ebenfalls reflektierend und für Ultraschall durchlässig ist.

In Fig. 7 ist schließlich ein Ausführungsbeispiel dargestellt, bei dem die Lichtapplikationsmittel 60 und die Ultraschallapplikationsmittel 24 ein Array aus wechselweise einer Mehrzahl von Lichtleiterelementen 62' und wechselweise einer Mehrzahl von Ultraschallwandlerelementen 20' aufweisen. Mit dieser Ausgestaltung des Applikationssystems kann ein zweidimensionales Schnittbild erzeugt werden, indem die einzelnen Arrayelemente mit einer bestimmten Phasenbeziehung phasengekoppelt angesteuert werden. Auf diese Weise wird ein elektronischer Abtastmechanismus anstelle des mit Bezug auf Fig. 1, 4 und 5 beschriebenen mechanischen Abtastmechanismus geschaffen.

## Patentansprüche

1. Bildgebendes Verfahren zum Ermitteln eines physikalischen oder chemischen Zustands von Gewebe (12) im menschlichen oder tierischen Körper unter Verwendung von Ultraschall, bei dem zumindest ein Ultraschallimpuls (16) im diagnostischen Frequenz- und Leistungsbereich in das Gewebe (12) eingekoppelt und der vom Gewebe (12) reflektierte Ultraschallechoimpuls (34) empfangen und in Ultraschallbildverarbeitungsmitteln (44) verarbeitet wird, wobei zumindest ein Lichtstrahl (52) erzeugt und in zumindest einen Meßlichtstrahl (58) und zumindest einen Referenzlichtstrahl (56) aufgespalten wird, der Meßlichtstrahl (58) entlang einer gleichen Strahlachse (64) wie der Ultraschallimpuls (16) in das Gewebe (12) eingekoppelt wird, und zwar so, daß der Meßlichtstrahl (58) und der zumindest eine Ultraschallimpuls (16) einander überlagert entlang der Strahlachse (64) in das Gewebe (12) eingekoppelt werden, der relative optische Weg zwischen dem Referenzlichtstrahl und dem Meßlichtstrahl verstellt wird, und der vom Gewebe rückgestreute Meßlichtstrahl (68) mit dem Referenzlichtstrahl (56) zur Interferenz gebracht und in optischen Bildverarbeitungsmitteln (84) verarbeitet wird .

2. Verfahren nach Anspruch 1, wobei der Lichtstrahl (52) mit einer spektralen Bandbreite im Bereich zwischen 10 und 200 nm erzeugt wird.

3. Verfahren nach Anspruch 1 oder 2, wobei der relative optische Weg zwischen dem Referenzlichtstrahl und dem Meßlichtstrahl über eine Kohärenzlänge des Lichts hinaus verstellt wird.

4. Verfahren nach einem der Ansprüche 1 bis 3, wobei eine kontinuierliche Folge von Ultraschallimpulsen (16) als Ultraschallstrahl in das Gewebe (12) eingekoppelt werden, wobei der Ultraschallstrahl und der Meßlichtstrahl (58) einander überlagert sind.

5. Verfahren nach Anspruch 4, wobei die gemeinsame Strahlachse (64) des Ultraschallstrahls und des Meßlichtstrahls (58) in einer Ebene parallel zur Oberfläche (26) des Gewebes (12) geschwenkt wird.

6. Verfahren nach Anspruch 4 oder 5, wobei die gemeinsame Strahlachse (64) des Ultraschallstrahls und des Meßlichtstrahls (58) um eine Drehachse quer zur momentanen Einstrahlrichtung verfahren wird.

7. Verfahren nach einem der Ansprüche 1 bis 6, wobei das durch die Verarbeitung des rückgestreuten Meßlichtstrahls (68) optisch erhaltene Bild und das durch Verarbeitung des Ultraschallechoimpulses (34) erhaltene Ultraschallbild miteinander derart kombiniert werden, daß im Nahbereich das optisch erhaltene Bild und im Fernbereich das Ultraschallbild dargestellt werden.

8. Verfahren nach einem der Ansprüche 1 bis 7, wobei mittels des durch die Verarbeitung des rückgestreuten Meßlichtstrahls (68) optisch erhaltenen Bildes die Dicke (d) einer oberflächennahen Gewebeschicht ermittelt wird, aus dem Ultraschallbild die Laufzeitdifferenz τ zwischen dem an einer ersten Gewebeschichtsgrenze (36) reflektierten Ultraschallechoimpuls (34) und dem an einer zweiten Gewebeschichtsgrenze (38) reflektierten Ultraschallechoimpuls (34) ermittelt wird, und aus der Laufzeitdifferenz τ und der Dicke d die Schallausbreitungsgeschwindigkeit c in der Gewebeschicht ermittelt wird.

9. Verfahren nach einem der Ansprüche 1 bis 8, wobei das Ultraschallbild als Übersichtsdarstellung des untersuchten Gewebes(12)und das optisch erhaltene Bild für eine Detaildarstellung ausgewählter Gewebebereiche herangezogen wird.

10. Verfahren nach einem der Ansprüche 1 bis 9, wobei zusätzlich durch den Meßlichtstrahl (58) oder durch unabhängig von diesem in das Gewebe (12) eingestrahltes Licht eine Fluoreszenz im Gewebe angeregt wird, und daß das Fluoreszenzlicht empfangen und das Fluoreszenzlichtbild zusätzlich zu dem optisch erhaltenen Bild dargestellt wird.

11. Bildgebendes System zum Ermitteln eines physikalischen oder chemischen Zustands von Gewebe (12) im menschlichen oder tierischen Körper unter Verwendung von Ultraschall, mit Ultraschallerzeugungsmitteln (14) zum Erzeugen zumindest eines Ultraschallimpulses (16) im diagnostischen Frequenz- und Leistungsbereich, mit Ultraschallapplikationsmitteln (24) zum Applizieren des Ultraschallimpulses (16) in das Gewebe (12), mit Ultraschallempfangsmitteln (32) zum Empfangen des vom Gewebe (12) reflektierten Ultraschallechoimpulses (34), und mit Ultraschallbildverarbeitungsmitteln (44) zum Verarbeiten des Ultraschallechoimpulses (34), weiterhin mit Lichterzeugungsmitteln (50) zum Erzeugen zumindest eines Lichtstrahles (52), Strahlteilermitteln (54) zum Aufspalten des Lichtstrahles (52) in zumindest einen Meßlichtstrahl (58) und zumindest einen Referenzlichtstrahl (56), Verstellmittel zum Verstellen des relativen optischen Weges zwischen dem Meßlichtstrahl (58) und dem Referenzlichtstrahl (56), Lichtapplikationsmitteln (60) zum Applizieren des Meßlichtstrahles (58) in das Gewebe (12), wobei die Lichtapplikationsmittel (60) und die Ultraschallapplikationsmittel (24) derart ausgebildet sind, daß der Ultraschallimpuls (16) und der Meßlichtstrahl (58) entlang einer gemeinsamen Strahlachse (64) einander überlagert in das Gewebe (12) einkoppelbar sind, Mitteln (66) zum Empfangen des vom Gewebe rückgestreuten Meßlichtstrahles (68) und Mitteln (70) zum interferometrischen Überlagern des rückgestreuten Meßlichtstrahles (68) mit dem Referenzlichtstrahl (56), sowie mit optische Bildverarbeitungsmitteln (84) zur Verarbeitung des interferometrischen Meßsignales.

12. System nach Anspruch 11, wobei die Lichterzeugungsmittel (50) eine Lichtquelle mit einer spektralen Bandbreite im Bereich zwischen 10 und 200 nm aufweisen.

13. System nach Anspruch 11 oder 12, wobei die Ultraschallapplikationsmittel (24) und die Lichtapplikationsmittel (60) zusammen in einem als Endoskop ausgebildeten Applikator (28) integriert sind.

14. System nach Anspruch 13, wobei die Ultraschallerzeugungsmittel (14) zumindest einen piezoelektrischen Ultraschallwander (20) aufweisen, und daß die Lichtapplikationsmittel (60) zumindest einen etwa mittig in einer Abstrahlfläche (30) des Ultraschallwandlers (20) endenden Lichtleiter (62) aufweisen.

15. System nach Anspruch 11 oder 10, wobei die Lichtapplikationsmittel (60) und die Ultraschallapplikationsmittel (24) eine Spiegelanordnung (92) aufweisen, die für Ultraschall durchlässig und für Licht reflektierend, oder umgekehrt, ist, um den Ultraschallimpuls (16) und den Meßlichtstrahl (58) entlang der gemeinsamen Strahlachse (64) einzukoppeln.

16. System nach einem der Ansprüche 11 bis 15, wobei die Strahlteilermittel (54) und die Mittel (70) zum interferömetrischen Überlagern des rückgestreuten Meßlichtstrahles (68) mit dem Referenzlichtstrahl (56) ein Zwei- oder Mehrstrahlinterferometer (74), bevorzugt ein Michelson-Interferometer, aufweisen.

17. System nach einem der Ansprüche 11 bis 16, weiter gekennzeichnet durch Mittel zum Verfahren der Strahlachse (64) in einer Ebene parallel zur Oberfläche (26) des Gewebes.

18. System nach einem der Ansprüche 11 bis 17, weiter gekennzeichnet durch Mittel zum Drehen der Strahlachse (64) um eine Drehachse quer zur momentanen Einstrahlrichtung.

19. System nach einem der Ansprüche 11 bis 18, wobei die Ultraschallbildverarbeitungsmittel (44) und die optischen Bildverarbeitungsmittel (84) miteinander derart gekoppelt sind, daß das Ultraschallbild und das optisch gewonnene Bild einander überlagert darstellbar sind.

20. Verwendung eines Verfahrens nach einem der Ansprüche 1 bis 10 und/oder eine Vorrichtung nach einem der Ansprüche 11 bis 19 zur Gewebedifferenzierung und/oder zur Ermittlung pathologischer Veränderungen in der Oberflächenstruktur von Gewebe.

21. Verwendung eines Verfahrens nach einem der Ansprüche 1 bis 10 und/oder eine Vorrichtung nach einem der Ansprüche 11 bis 19 zur sichtbaren Darstellung dynamischer Prozesse, beispielsweise einem Blutfluß oder Bewegungsabläufen im Gewebe.

## Claims

1. An imaging method for determining a physical or chemical condition of tissue (12) in a human or animal body using ultrasound, where at least one ultrasonic pulse (16) in the diagnostic frequency and power range is injected into the tissue (12), and the ultrasonic echo pulse (34) reflected by the tissue (12) is received and processed in ultrasonographic image processing means (44), **wherein** at least one light beam (52) is generated and split up into at least one measuring light beam (58) and at least one reference light beam (56), the measuring light beam (58) is injected along the same beam axis (64) along which the ultrasonic pulse (16) is injected into the tissue (12), such that the measuring light beam (58) and the at least one ultrasonic pulse (16) are injected into the tissue (12) along the beam axis in superimposed fashion, the relative optical path between the reference light beam and the measuring light beam is adjusted, and the measuring light beam (68) scattered back by the tissue is brought into an interference relationship with the reference light beam (56) and is processed in optical image processing means (84).

2. The method of claim 1, wherein the light beam (52) is generated with a spectral bandwidth in a range of between 10 and 200 nm.

3. The method of claim 1 or 2, wherein the relative optical path between the reference light beam and the measuring light beam is adjusted beyond one coherence length of the light.

4. The method of one of claims 1 through 3, wherein a continuous sequence of ultrasonic pulses (16) is injected into the tissue (12) as ultrasonic beam, the ultrasonic beam and the measuring light beam (58) being superimposed.

5. The method of claim 4, wherein the common beam axis (64) of the ultrasonic beam and the measuring light beam (58) are swept in a plane parallel to the surface (26) of the tissue (12).

6. The method of claim 4 or 5, wherein the common beam axis (64) of the ultrasonic beam and of the measuring light beam (58) is rotated about a rotary axis transversely to the instantaneous direction of irradiation.

7. The method of one of claims 1 through 6, wherein the image obtained optically by processing the back-scattered measuring light beam (68) and the ultrasonographic image obtained by processing the ultrasonic echo pulse (34) are combined one with the other so that the image optically obtained is displayed in the near range and the ultrasonographic image is displayed in the far range.

8. The method of one of claims 1 through 7, wherein the thickness (d) of a tissue layer near the surface is determined by means of the optical image obtained by processing the back-scattered measuring light beam (68), the propagating time delay τ between the ultrasonic echo pulse (34) reflected at a first tissue layer boundary (36) and the ultrasonic echo pulse (34) reflected at a second tissue layer boundary (38) is determined from the ultrasonographic image, and the sound propagation speed c in the tissue layer is determined from the propagating time delay τ and the thickness d.

9. The method of one of claims 1 through 8, wherein the ultrasonographic image is used as overview image of the tissue (12) being examined, while the optical image is used for the detailed imaging of selected tissue regions.

10. The method of one of claims 1 through 9, wherein fluorescence is additionally stimulated in the tissue by the measuring light beam (58) or by light irradiated into the tissue (12) independently thereof, and the fluorescent light is received, and the fluorescent light image is displayed in addition to the image optically obtained.

11. Imaging system for determining a physical or chemical condition of tissue (12) in a human or animal body using ultrasound, comprising ultrasound-generating means (14) for generating at least one ultrasonic pulse (16) in the diagnostic frequency and power range, ultrasound application means (24) for applying the ultrasonic pulse (16) into the tissue (12), ultrasound receiving means (32) for receiving the ultrasonic image pulse (34) reflected by the tissue (12), and ultrasonographic image processing means (44) for processing the ultrasonic echo pulse (34), further comprising light generating means (50) for generating at least one light beam (52), beam splitter means (54) for splitting up the light beam (52) into at least one measuring light beam (58) and at least one reference light beam (56), adjusting means for adjusting the relative optical path between the measuring light beam (58) and the reference light beam (56), light application means (60) for applying the measuring light beam (58) into the tissue (12), the light application means (60) and the ultrasound application means (24) being designed in such a way that the ultrasonic pulse (16) and the measuring light beam (58) are injected into the tissue along a common beam axis (64) in superimposed fashion, further means (66) for receiving the measuring light beam (68) scattered back by the tissue and means (70) for interferometrically superimposing the back-scattered measuring light beam (68) and the reference light beam (56), as well as optical image processing means (84) for processing the interferometric measuring signal.

12. The system of claim 11, wherein the light-generating means (50) comprise a light source having a spectral bandwidth in the range of between 10 and 200 nm.

13. The system of claim 11 or 12, wherein the ultrasound application means (24) and the light application means (60) are both integrated in an applicator (28) designed as endoscope.

14. The system of claim 13, wherein the ultrasound generating means (14) comprise at least one piezoelectric ultrasonic transducer (20), and the light application means (60) comprise at least one light pipe (62) ending substantially centrally in a radiation surface (30) of the ultrasonic transducer (20).

15. The system of claim 11 or 10, wherein the light application means (60) and the ultrasound application means (24) comprise a mirror arrangement (92) that is permeable to ultrasound and reflecting to light, or vice versa, in order to inject the ultrasonic pulse (16) and the measuring light beam (58) along the common beam axis (64).

16. The system of one of claims 11 through 15, wherein the beam splitter means (54) and the means (70) for interferometrically superimposing the back-scattered measuring light beam (68) and the reference light beam (56) comprise a double-beam or multiple-beam interferometer (74), preferably a Michelson interferometer.

17. The system of one of claims 11 through 16, further characterized by means for sweeping the beam axis (64) in a plane parallel to the surface (26) of the tissue.

18. The system of one of claims 11 through 17, further characterized by means for rotating the beam axis (64) about a rotary axis transversely to the instantaneous direction of irradiation.

19. The system of one of claims 11 through 18, wherein the ultrasonic image processing means (44) and the optical image processing means (84) are coupled one with the other in such a way that the ultrasonographic image and the image optically obtained can be displayed in superimposed fashion.

20. Use of a method of one of claims 1 through 10 and/or of a system of one of claims 11 through 19 for tissue differentiation and/or for determining pathological changes in the surface structure of tissue.

21. Use of a method of one of claims 1 through 10 and/or of a system of one of claims 11 through 19 for visualizing dynamic processes, such as a blood flow or motions in the tissue.

## Revendications

1. Procédé graphique destiné à déterminer un état physique ou chimique d'un tissu (12) dans le corps humain ou animal, par utilisation d'ultrasons, dans lequel au moins une impulsion d'ultrasons (16) dans la gamme de fréquences et de puissances de diagnostique est envoyée dans le tissu (12) et l'impulsion d'écho d'ultrasons (34), réfléchie par le tissu (12) est reçue et est traitée dans des moyens (44) de traitement d'images aux ultrasons, dans lequel au moins un rayon lumineux (52) est produit et est divisé en au moins un rayon lumineux de mesure (58) et au moins un rayon lumineux de référence (56), le rayon lumineux de mesure (58) est envoyé dans le tissu (12), le long d'un même axe de rayon (64) que l'impulsion d'ultrasons (16), et ce de manière que le rayon lumineux de mesure (58) et la ou les impulsions d'ultrasons (16) soit(soient) envoyée(s) superposées dans le tissu (12), le long de l'axe de rayon (64), la distance optique relative entre le rayon lumineux de référence et le rayon lumineux de mesure est réglée, et le rayon lumineux de mesure (68) redispersé par le tissu est amené en interférence avec le rayon lumineux de référence (56) et est traité dans des moyens optiques (84) de traitement d'images.

2. Procédé selon la revendication 1, dans lequel le rayon lumineux (52) est produit avec une largeur de bande spectrale comprise entre 10 et 200 nm.

3. Procédé selon la revendication 1 ou 2, dans lequel la distance optique relative entre le rayon lumineux de référence et le rayon lumineux de mesure est déplacée au-delà d'une longueur de cohérence de la lumière.

4. Procédé selon l'une des revendications 1 à 3, dans lequel une succession continue d'impulsions d'ultrasons (16) est envoyée dans le tissu (12) en tant que rayons d'ultrasons, le rayon d'ultrasons et le rayon lumineux de mesure (58) étant superposés l'un à l'autre.

5. Procédé selon la revendication 4, dans lequel l'axe (64) commun du rayon d'ultrasons et du rayon lumineux de mesure (58) est pivoté dans un plan parallèlement à la surface (26) du tissu (12).

6. Procédé selon la revendication 4 ou 5, dans lequel l'axe (64) commun du rayon d'ultrasons et du rayon lumineux de mesure (58) est déplacé autour d'un axe de rotation, perpendiculairement à la direction à l'instant du rayonnement incident.

7. Procédé selon l'une des revendications 1 à 6, dans lequel l'image obtenue optiquement par le traitement du rayon lumineux de mesure (68) redispersé et l'image d'ultrasons, obtenue par traitement de l'impulsion d'écho d'ultrasons (34) sont combinées de manière que dans le domaine proche soit représentée l'image obtenue optiquement et dans le domaine lointain, l'image d'ultrasons.

8. Procédé selon l'une des revendications 1 à 7, dans lequel l'épaisseur (d) d'une couche de tissu proche de la surface est déterminée au moyen de l'image obtenue optiquement par traitement du rayon lumineux de mesure (68) redispersé, la différence de durée (τ) entre l'impulsion d'écho d'ultrasons (34) réfléchie sur une première limite (36) de couches de tissu et l'impulsion d'écho d'ultrasons (34), réfléchie sur une deuxième limite (38) de couches de tissu, est déterminée à partir de l'image d'ultrasons, et la vitesse (c) de propagation du son dans la couche de tissu est déterminée à partir de la différence de durée (τ) et l'épaisseur (d).

9. Procédé selon l'une des revendications 1 à 8, dans lequel l'image d'ultrasons est utilisée en tant que représentation d'ensemble du tissu (12) analysé et l'image obtenue optiquement est utilisée pour une représentation de détails de zones choisies du tissu.

10. Procédé selon l'une des revendications 1 à 9, dans lequel en supplément une fluorescence est générée dans le tissu, par le rayon lumineux de mesure (58) ou par une lumière rayonnée indépendamment de celui-ci dans le tissu (12), et la lumière fluorescente est reçue et l'image de lumière fluorescente est représentée en supplément à l'image obtenue optiquement.

11. Système imagé destiné à déterminer un état physique ou chimique d'un tissu (12) dans le corps humain ou animal par utilisation d'ultrasons, comportant des moyens (14) de génération d'ultrasons destinés à produire au moins une impulsion d'ultrasons (16) dans la gamme de fréquences et de puissances de diagnostique, comportant des moyens (24) d'application d'ultrasons destinés à appliquer l'impulsion d'ultrasons (16) dans le tissu (12), comportant des moyens (32) de réception d'ultrasons pour la réception de l'impulsion d'écho d'ultrasons (34) réfléchie par le tissu (12), et comportant des moyens (44) de traitement de l'image d'ultrasons pour le traitement de l'impulsion d'écho d'ultrasons (34), comportant en outre des moyens (50) de production de lumière pour produire au moins un rayon lumineux (52), des moyens diviseurs de rayons (54) pour diviser le rayon lumineux (52) en au moins un rayon lumineux de mesure (58) et au moins un rayon lumineux de référence (56), des moyens de déplacement pour déplacer la distance optique relative entre le rayon lumineux de mesure (58) et le rayon lumineux de référence (56), des moyens d'application de lumière (60) et des moyens d'application d'ultrasons (24) étant conçus de manière que l'impulsion d'ultrasons (16) et le rayon lumineux de mesure (58) puissent être envoyés dans le tissu (12), superposés l'un à l'autre le long d'un axe de rayon (64) commun, des moyens (66) pour la réception du rayon lumineux de mesure (68) redispersé par le tissu et des moyens (70) pour la superposition interférométrique du rayon lumineux de mesure (68) redispersé et du rayon lumineux de référence (56), et comportant des moyens optiques (84) de traitement de l'image pour le traitement du signal de mesure interférométrique.

12. Système selon la revendication 11, dans lequel les moyens de production de lumière (50) comportent une source lumineuse d'une largeur de bande spectrale comprise entre 10 et 200 nm.

13. Système selon la revendication 11 ou 12, dans lequel les moyens d'application d'ultrasons (24) et les moyens d'application de lumière (60) sont intégrés dans un applicateur (28) réalisé en tant qu'endoscope.

14. Système selon la revendication 13, dans lequel les moyens de production d'ultrasons (14) comportent au moins un convertisseur d'ultrasons (20) piézoélectrique, et les moyens d'application de lumière (60) comportent au moins un guide de lumière (62) qui se termine à peu près au milieu d'une surface de rayonnement (30) du convertisseur d'ultrasons (20).

15. Système selon la revendication 10 ou 11, dans lequel les moyens d'application de lumière (60) et les moyens d'application d'ultrasons (24) comportent un ensemble de miroirs (92), qui laisse passer les ultrasons et réfléchit la lumière, ou inversement, afin d'envoyer l'impulsion d'ultrasons (16) et le rayon lumineux de mesure (58) le long de l'axe de rayon (64) commun.

16. Système selon l'une des revendications 11 à 15, dans lequel les moyens diviseurs de rayons (54) et les moyens (70) pour la superposition interférométrique du rayon lumineux de mesure (68) redispersé et du rayon lumineux de référence (56), comportent un interféromètre (74) à deux rayons ou plus, de préférence, un interféromètre de Michelson.

17. Système selon l'une des revendications 11 à 16, caractérisé en outre par des moyens permettant de déplacer l'axe de rayon (64) dans un plan, parallèlement à la surface (26) du tissu.

18. Système selon l'une des revendications 11 à 17, caractérisé en outre par des moyens destinés à faire tourner l'axe de rayon (64) autour d'un axe de rotation transversalement à la direction momentanée du rayonnement incident.

19. Système selon l'une des revendications 11 à 18, dans lequel les moyens (44) de traitement de l'image d'ultrasons et les moyens (84) de traitement de l'image optique sont couplés entre eux de manière que l'image d'ultrasons et l'image obtenue optiquement puissent être représentées superposées l'une à l'autre.

20. Mise en oeuvre d'un procédé selon l'une des revendications 1 à 10 et/ou utilisation d'un dispositif selon l'une des revendications 11 à 19 pour la différenciation de tissus et/ou pour la détermination de variations pathologiques dans la structure superficielle d'un tissu.

21. Mise en oeuvre d'un procédé selon l'une des revendications 1 à 10 et/ou utilisation d'un dispositif selon l'une des revendications 11 à 19 pour la représentation visible de processus dynamiques, par exemple d'un flux sanguin ou de mouvements dans le tissu.
